# EUROPEAN PATENT SPECIFICATION

(11) **EP 3 927 231 B1**
(45) Date of publication and mention of the grant of the patent: **28.08.2024**
(21) Application number: 20919956.1
(22) Date of filing: 14.09.2020
(51) Int. Cl.: A61H 9/00, A61B 5/00, A61B 5/02, A61B 5/022, A61B 5/024, A61B 5/0225

(54) **HIGH EFFICIENCY EXTERNAL COUNTER PULSATION SYSTEM AND METHOD OF TREATMENT USING THE SYSTEM**
HOCHEFFIZIENTES EXTERNES GEGENPULSATIONSSYSTEM UND BEHANDLUNGSVERFAHREN MIT DEM SYSTEM
SYSTÈME DE CONTRE-PULSATION EXTERNE À EFFICACITÉ ÉLEVÉE ET PROCÉDÉ DE TRAITEMENT UTILISANT LE SYSTÈME

(30) Priority: 20.02.2020 US 202062979372 P
(43) Date of publication of application: 29.12.2021
(73) Proprietor: Academia Sinica, Taipei 11529 (TW)
(72) Inventor: YANG, Fu-Liang, Hsinchu City, 30080 (TW); CHUNG, Chang-Kuei, Taoyuan City, 338 (TW)
(74) Representative: Charrier Rapp & Liebau
(86) International application number: PCT/US2020/050606
(87) International publication number: WO 2021/167651

(56) References cited:
- CN-A- 110 279 574
- US-A- 3 454 010
- US-A- 6 010 471
- US-A- 6 010 471
- US-A1- 2010 160 795
- US-A1- 2013 102 939
- US-A1- 2013 102 939
- US-A1- 2013 116 582
- US-B2- 10 010 744

## Description

### Field of the Invention

The present invention relates to a highly efficient external counter pulsation system according to the preamble portion of claim 1. Specifically, the present invention comprises one or more air bladders that utilize helical geometry of the major veins and arteries in users' thigh to achieve high efficiency.

### Background of the Invention

US 2013/102939 A1 discloses an external counter pulsation device comprising: an air bladder system comprising an air bladder and one adjunct air bladder; a valve and fluid system pneumatically connected to the air bladder system wherein the valve and fluid system is configured to pressurize and depressurize the air bladder and the adjunct air bladders; and a control system comprising a processor and one PPG sensor and one ECG sensor wherein the PPG and ECG sensors are connected to the user to collect PPG and ECG signals from the user and wherein the control system is electronically connected to the valve and fluid system to control the valve and fluid system to pressurize or depressurize the air bladders of the air bladder system based on signals detected by the sensors and wherein the adjunct air bladders is positioned at either the lower end or the upper end of the air bladder and is pressurized before the air bladder so as to direct blood flow towards desired direction.

External Counter Pulsation (ECP) is a clinically proven treatment system for various diseases such as refractory angina, acute myocardial infarction, congestive heart failure and ischemia related diseases by using air bladders on the leg to modulate hemodynamic characteristics. Other applications are currently being explored in neurology and nephrology. However, current ECP systems are expensive, large, heavy and stationary. One reason is that high powered air compressors are required to operate the systems. Therefore, only hospitals and clinics are able to purchase and house them, requiring patients to travel to receive ECP treatments.

The design of the air bladders can substantially influence efficiency of an ECP system, including machine dimension and electrical power consumption. This invention discloses a novel helix air bladder-based high efficiency ECP system, in which the helix air bladder takes advantage of the helical manner that the major arteries and veins in the thigh winds around the femur to efficiently modulate blood flow by pressing on the major arteries and veins against the femur. Therefore the artery will be pressed by both the action force of helix air bladder and by the reaction force of the femur to make most use of applied air pressure. We add an adjunct air bladder on one end of the helix air bladder to further confine the pressed artery blood moving towards the desired direction. Special cuffs to accommodate the air bladders are designed to ensure high air pressure transfer efficiency to artery. The invention discloses the whole air piping loop and the relevant control method to realize a high efficiency ECP system. The efficiency realized by the present invention using the novel helical air bladders substantially reduces air compressor power requirements and thereby reduces the cost as well as size and weight of the ECP system of the present invention so that owning and running the ECP system of the present invention in house is possible.

### Summary of the Invention

The present invention relates to an external counter pulsation (ECP) device comprising the subject features of claim 1.

In another embodiment, helix air bladder's length L in cm is defined as height of the user/3.2 -b where b is between 15 cm to 30 cm, helix air bladder's top width and helix air bladder's lower width are about 14 cm and helix angle is about 55°. In yet another embodiment, the wattage of the valve system is less than about 1500 Watts.

In an embodiment, the helix air bladder length L does not exceed 50 cm, W1 and W2 do not exceed 25 cm and 1250 cm² in area. In another embodiment, the pressure within the helix air bladder does not exceed 350 mmHg when fully pressurized. In another embodiment, the pressure of the helix air bladder is not below about 150 mmHg when fully pressurized. In yet another embodiment, the ratio of the top width of the helix air bladder W1 to the bottom width W2 of the helix air bladder is about from 1:1 to 2:1.

In an embodiment, the helix angle of the helix air bladder is between about 30° and 75°. In another embodiment, the adjunct air bladder is positioned at the lower end of the helix air bladder with the adjunct air bladder overlapping the helix air bladder. In yet another embodiment, the adjunct air bladder is positioned at the lower end of the helix air bladder without the adjunct air bladder overlapping the helix air bladder.

In an embodiment, the adjunct air bladder is positioned at the upper end of the helix air bladder with the adjunct air bladder overlapping the helix air bladder. In another embodiment, the adjunct air bladder is positioned at the upper end of the helix air bladder without the adjunct air bladder overlapping the helix air bladder. In yet another embodiment, the adjunct air bladder and the helix air bladder are in one single cuff.

A method for providing external counter pulsation treatment using ECP device of the present invention comprising the steps of detecting R peak of a user's heartbeat, instituting a delay of about 10 ms to 250 ms from the R peak, pressurizing the adjunct air bladder, instituting a delay of about 20 ms to 100 ms, pressurizing the helix air bladder for a therapeutically effective amount of time of about 200 ms to 600 ms, depressurizing both the adjunct air bladder and helix air bladder at about the same time, and repeating steps a-f for a therapeutically effective amount of time, is not part of the invention.

### Brief Description of the Drawings

**Figure 1** is a diagram illustrating the helical geometry of major arteries and veins in the thigh of a user and an embodiment of the helix air bladder of the present invention 10.
**Figure 2** is a high level block diagram of the present invention 10.
**Figure 3** is a detailed diagram of an embodiment of helix cuff 105 and adjunct cuff 160 of the present invention.
**Figures 4a and 4b** are diagrams illustrating two different possible placements of the helix cuff 105 and adjunct cuff 160 of the present invention.
**Figures 4c and 4d** are diagrams illustrating two different possible placements of the helix air bladder 110 and adjunct air bladder 170 when they are both constructed within one cuff.
**Figures 5a and 5b** are diagrams showing the pressurization and depressurization cycles of the ECP device of the present invention 10 as they relate to cardiac cycles and blood flow when the adjunct air bladder 170 is located at the lower end of the helix air bladder 110.
**Figures 6a and 6b** are diagrams showing the pressurization and depressurization cycles of the ECP device of the present invention 10 as they relate to cardiac cycles and blood flow when the adjunct air bladder 170 is located at the upper end of the helix air bladder 110.
**Figure 7** is a block diagram illustrating an embodiment of the ECP control unit 200 of the present invention.
**Figure 8** is a block diagram depicting an embodiment of the ECP control unit 200 and the fluid and valve system 300 of the present invention.
**Figure 9** is a block diagram depicting an embodiment of the fluid and valve system 300 of the present invention.
**Figure 10** is a flow chart illustrating an embodiment to of a method of using the ECP device of the present invention 10.
**Figure 11** is a combination of PPG and ECG charts with charts showing pressurization and depressurization of helix air bladder 110 and adjunct air bladder 170 wherein the diagrams are synched in time to illustrate a method of treatment using the ECP device of the present invention 10.
Figures 12a and 12b illustrate an exemplary PPG and ECG signals of a user before and during treatment using the ECP device of the present invention 10, respectively.

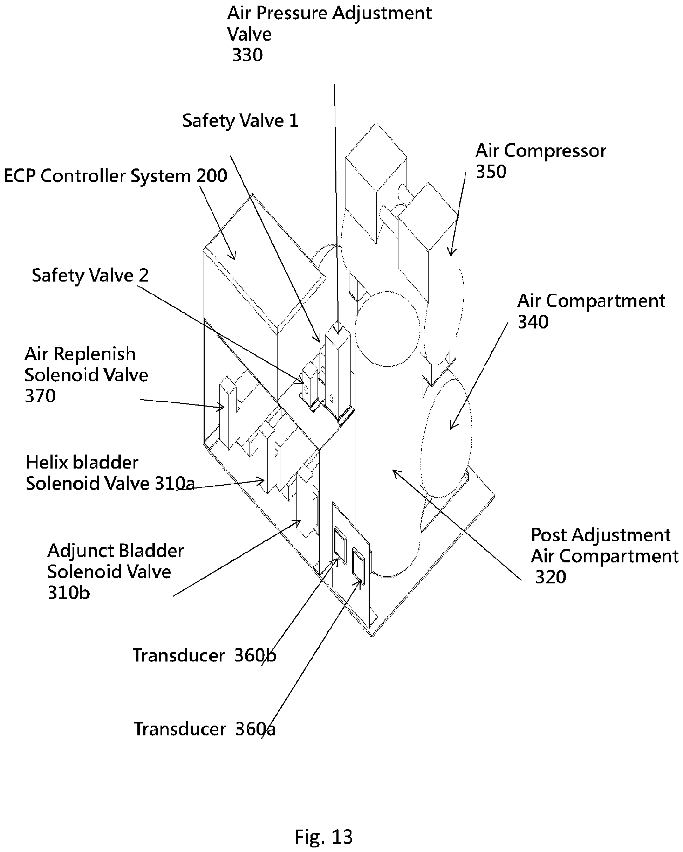

**Figure 13** is a drawing of an exemplary embodiment of the ECP control system 200 and fluid and valves system 300 of the ECP device of the present invention 10.

### Detailed Description of the Present Invention

As used in this specification and in claims which follow, the singular forms "a", "an" and "the" include plural referents unless the context clearly indicates otherwise. Thus, for example, reference to "an ingredient" includes mixtures of ingredients, reference to "an active pharmaceutical agent" includes more than one active pharmaceutical agent, and the like.

As used herein, the term "about" as a modifier to a quantity is intended to mean + or - 5% inclusive of the quantity being modified.

The term "effective amount of time" or "a therapeutically effective amount of time" of a treatment is intended to mean a nontoxic/unharmful but sufficient amount of time required for providing the desired therapeutic effect. The time period that is "effective" may vary from subject to subject, depending on the age and general condition of the individual, the particular conditions, and the like.

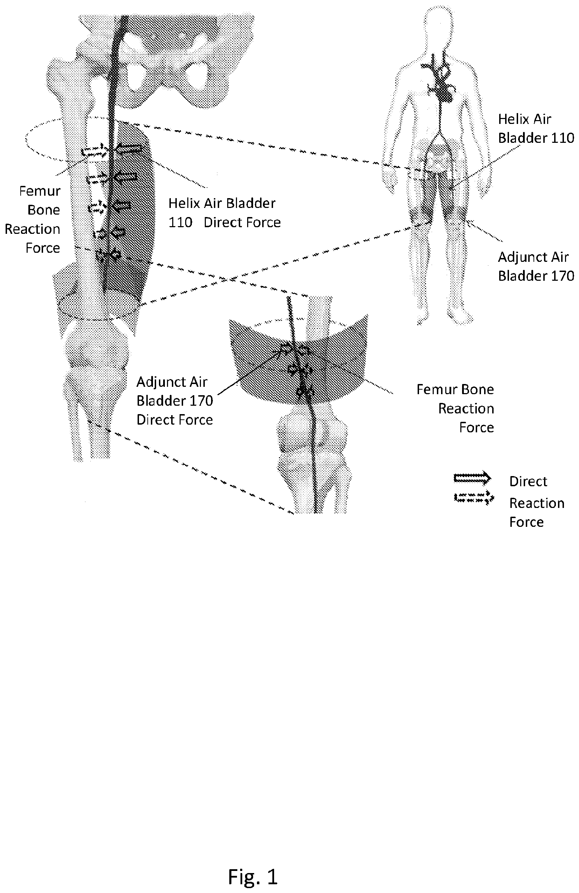

The ECP device 10 of the present invention is capable of achieving miniaturization, low energy consumption and low device cost as compared to prior art ECP devices. This is possible because the ECP device 10 of the present invention takes advantage of the geometry of major arteries and veins in the thigh which wind around the femur in a helical manner shown in figure 1. Specifically, as shown in figure 1, the major arteries and veins start in front of the femur in front of the pelvic bone, winds around the femur in a helical fashion towards the inner thigh and ends up behind the femur behind the knee before travelling farther down the leg. As shown in figure 1, the helix air bladder 110 is specifically shaped to take advantage of this particular anatomy so that air bladder 110 focuses its energy only on the area of the thigh required to press the major veins and arteries against the femur bone in order to modulate blood flow of the major arteries and veins while wasting little energy on other areas of the thigh. The efficiency achieved means that a much smaller and less powerful air compressor is required for achieving the same or better therapeutic results compared to traditional ECP. For example, the power consumption of the present invention is below about 500, 600, 700, 800, 900, 1000, 1250 or 1500 Watts as compared to around 2500 Watts energy consumption of the typical commercial units currently available. In addition, the miniaturization renders the ECP device of the present invention 10 easy to handle, even portable, at less than about 20, 25 or 30 kg, and cost substantially less than existing ECP devices which are typically so heavy that they are made to be stationary. This means that users may easily own and operate the ECP device of the present invention 10 at home rather than having to travel to a clinic for treatment.

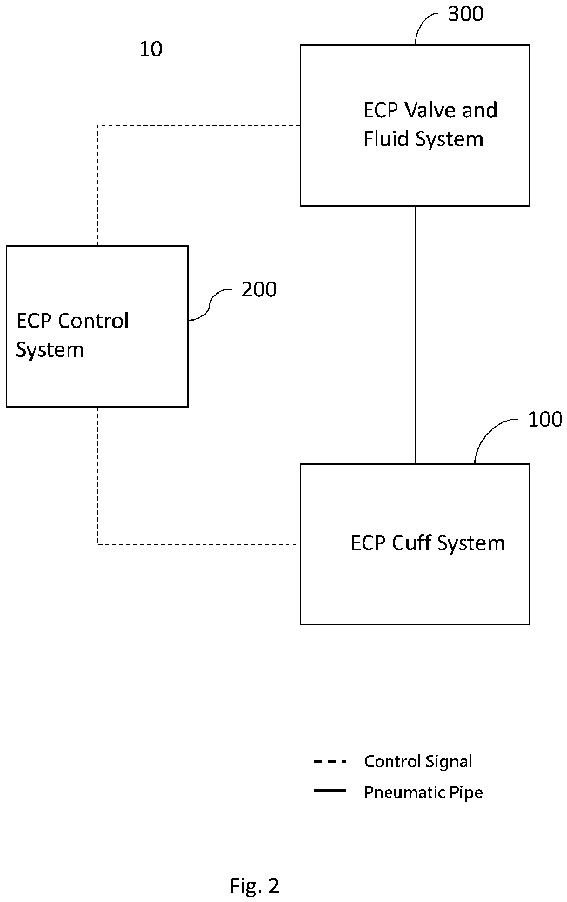

Figure 2 is a high level depiction of the ECP device 10 of the present invention 10 comprising an air bladder system 100, a control system 200 and a valve and fluid system 300.

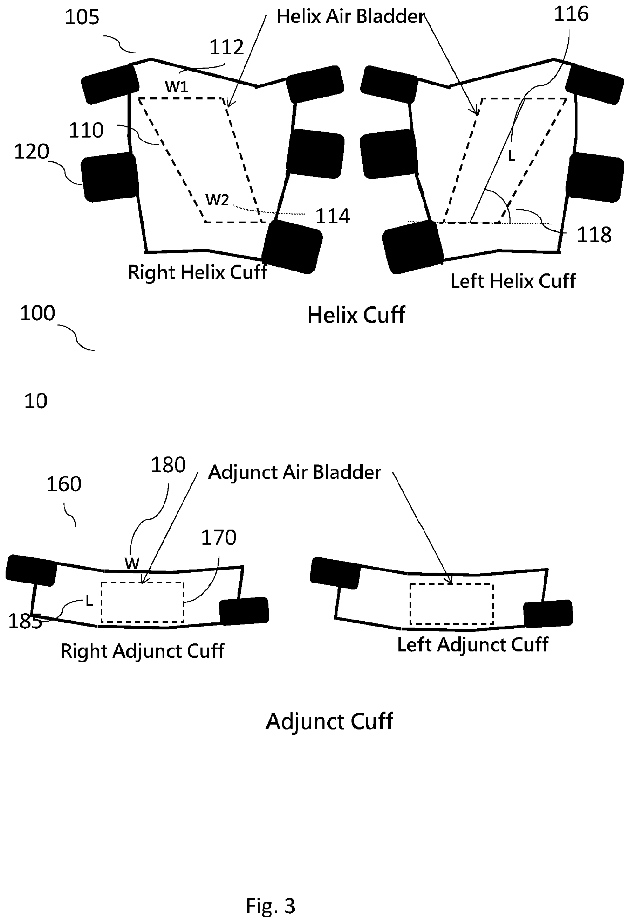

Figure 3 depicts an embodiment of the cuff system 100. As shown in figure 3, the helix cuff system 100 comprises a helix cuff 105 and a helix air bladder 110. In an embodiment, the helix air bladder 110 comprises an upper width W1 112, lower width W2 114, length L 116 and helix angle 118, where length L 116 is the straight line between the midpoint of width W1 112 and midpoint of width W2 114. Helix angle is the angle between length L 116 and horizontal line parallel to the level ground when the cuff is worn by the user standing upright. If W2 is designed as parallel to this horizontal line as illustrated in figure 3, the helix angle would be the angle between width W2 and length L 116. In another embodiment, the helix cuff 105 further comprises one or more cuff fasteners 120.

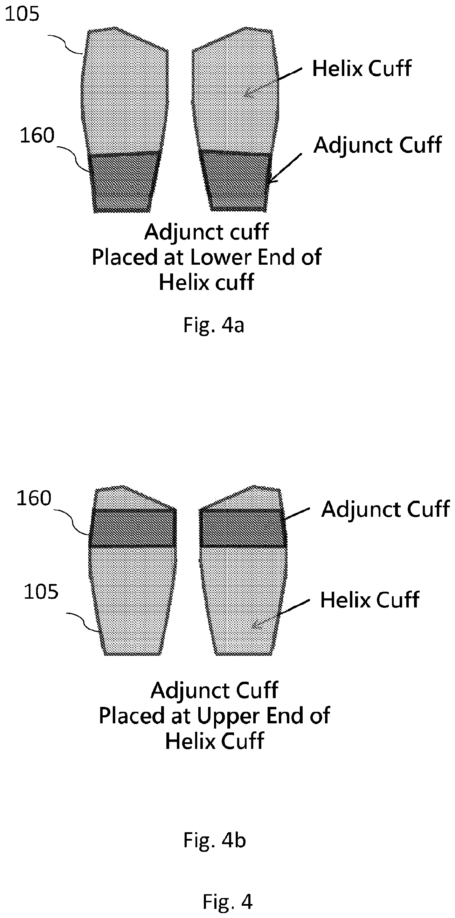

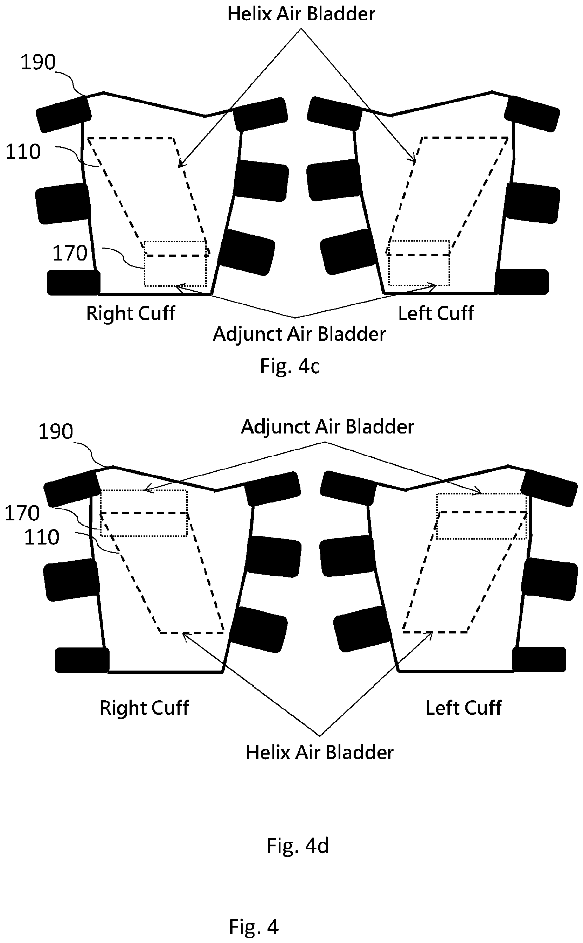

In an embodiment as described above, the helix air bladder 110 is placed over major artery and or vein of the inner thigh such that the helix shape of the air bladder 110 follows the major arteries and veins that wrap around the femur as shown in figure 1. Upper width W1 112, lower width W2 114, length L 116 and helix angle 118 may differ depending on factors such as location of placement and biometrics of the user such as sex, height, weight, BMI, age, etc... in order to better conform to the major arteries and or veins around the femur. In one embodiment, the helix angle 118 is about between 30 ° to 75 °, 40 ° to 65 ° or about 55 °. In one embodiment, the helix air bladder's upper width W1 112 is wider than the lower width W2 114 as shown in figure 3. In another embodiment, the ratio of W1 112 :W2 114 is between about 2:1 to 1:1, about 1.9:1 to 1.1:1, about 1.8 to 1.2:1, about 1.7:1 to 1.3:1, about 1.6:1 to 1.4:1 or about 1.5:1. The fact that W1 112 is wider than W2 114 helps to direct the blood upwards towards the torso when the helix air bladder 110 is inflated as shown in fig.4a and 4c. In one embodiment, the ratio of the width W1 112 of the top of the helix air bladder 110 to the length of the air bladder L 116 is about 1:2 to 1:4 or about 1:3.

In one embodiment, the helix air bladder 110 only covers the thigh area. In another embodiment, the helix air bladder 110 does not cover the entire thigh but just enough area over the major artery or vein over the femur as necessary to therapeutically effectively modulate blood flow so that the cuff system 100 and ECP device 10 overall may be miniaturized. In an embodiment, L 116, W1 112 and W2 114 are dependent on the biometrics of a user such as height and/or weight of the user. For example, in an embodiment: L= (User Height/3.2) - b where b is between about 15 cm to about 30 cm. So that if user height is 165 cm, L can be between about 21.6 cm and 36.6 cm and W1 112 is about 14 cm and W2 114 is about 14 cm.

In one embodiment, as shown in fig. 3, the ECP device of the present invention 100 further comprises an adjunct cuff 160. In an embodiment, the adjunct cuff 160 comprises an adjunct air bladder 170 which assists the helix air bladder 110 in modulating blood flow towards or away from the user's torso as will be described in further detail below in connection with figs. 4-6. As with the design considerations for the helix air bladder 110, width W 180 and length L 185 of the adjunct air bladder 170 should be minimized to reduce power and size requirements of the air compressor used to pressurize it but still provide adequate assistance to the helix air bladder 110 as described further in connection with figure 4. However, the width W 180 of the adjunct air bladder 170 should be wide enough to fully encompass the W2 114 width of the helix air bladder 110 if the adjunct cuff 160 is placed at the lower end of the helix cuff 105 as illustrated on figs. 4a and c or the W1 width 112 if the adjunct cuff 160 is placed at the upper end of the helix cuff 105 as illustrated on figs. 4b and d. In addition, the length L 185 and width W 180 of the adjunct air bladder 170 should be sized and the pressure provided by the air compressor should be high enough to provide adequate force in assisting the helix air bladder 110 in modulating blood flow towards the desired direction as described in different configurations below in connection with figure 4. In one embodiment, the ratio of the width W 180 to length L 185 of the adjunct air bladder 170 is about 1.5:1 to 4:1, about 2:1 to 3:1 or about 2.5:1. In another embodiment, the width W 180 of the adjunct air bladder is about 8 cm to 30 cm, about 16 to 24 cm or about 22 cm.

In one embodiment as illustrated in figures 4a-d, the adjunct air bladder 170 may be positioned over the helix air bladder 110 in different configurations. In one embodiment, the adjunct air bladder 170 may be positioned over the lower end of the helix air bladder 110 as shown in figure 4a. In an embodiment, the adjunct air bladder 170 abuts the lower end of the helix air bladder at the bottom of W2 114 and overlaps the helix air bladder 110. In yet another embodiment, the helix air bladder 110 and adjunct air bladder 170 may be built in one single cuff as shown in fig. 4c.

In another embodiment, as shown in fig. 4b, the adjunct air bladder 170 may be positioned over the upper end of the helix air bladder 110. In an embodiment, the adjunct air bladder 170 abuts the upper end of the helix air bladder at the top of W1 112 and overlaps the helix air bladder 110. In another embodiment, the helix air bladder 110 and adjunct air bladder 170 may be built in one single cuff as shown in fig. 4d.

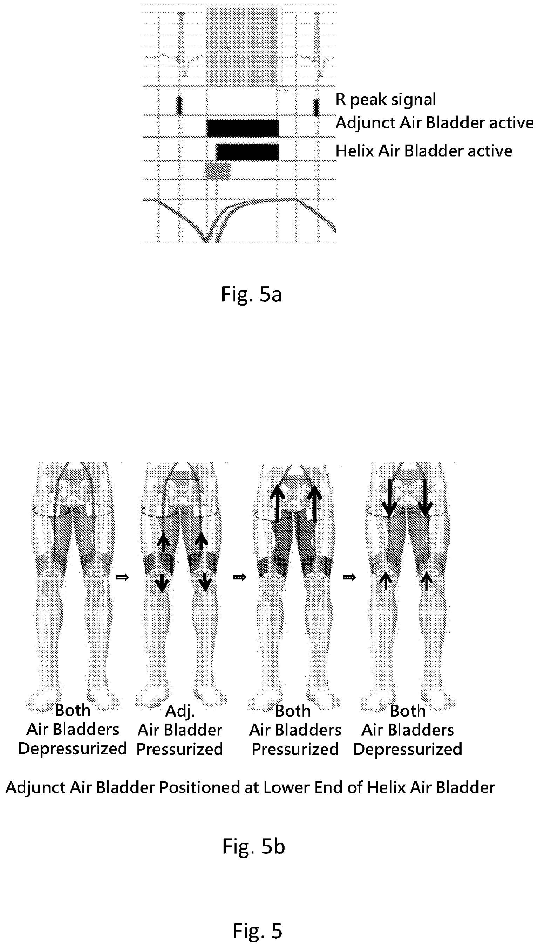

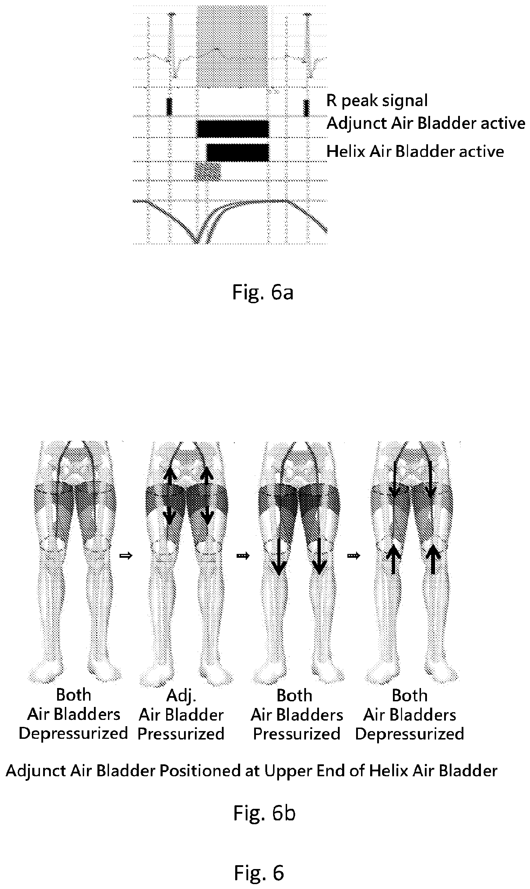

In an embodiment, the adjunct air bladder 170 is pressurized before the helix air bladder 170 so as to affect direction of blood flow when the helix air bladder 110 is subsequently pressurized. Specifically, as shown in fig. 5, when the adjunct air bladder 170 is positioned at the lower end of the helix air bladder 110 and the adjunct air bladder 170 is pressurized before the helix air bladder 110, the direction of the majority of blood flow caused by the cuff system 100 is first upwards towards the torso of the user since adjunct air bladder 170 prevents downward blood flow. In an embodiment, the adjunct air bladder 170 should be wide enough to at least cover the entire width W1 112 of the helix air bladder. In addition, air pressure of adjunct air bladder 170 should be high enough to stop over about 90%, about 80%, about 70% or about 60% of the blood flow downwards when pressurized. In an embodiment, the pressure in the air bladders 110 and 170 is about 150 mmHg to 350 mmHg, about 200 mmHg to 300 mmHg or about 250 mmHg. Subsequently, when both air bladders 170 and 110 depressurize, majority of blood flow caused by the cuff system 100 is downwards away from the torso of the user. And in figure 6 in an embodiment in which the adjunct air bladder 170 is positioned at the upper half of the helix air bladder 110 and when the adjunct air bladder 170 is pressurized before the helix air bladder 110, the direction of the majority of blood flow caused by the cuff system 100 is first downwards towards the feet of the user since adjunct air bladder 170 prevents upward blood flow. Subsequently, when both air bladders 170 and 110 depressurize at about the same time, the majority of blood flow caused by the cuff system 100 is upwards towards the torso of the user. Therefore, air pressure of the adjunct air bladder 170 should be high enough to stop over 90%, about 80%, about 70% or about 60% of the blood flow upwards when pressurized. In an embodiment, the pressure in the air bladders 110 and 170 is about 150 mmHg to 350 mmHg, 200 to 300 or about 250 mmHg. As illustrated in figures 5 and 6, placement of the air bladders 110 and 170 allows the user to target different areas of the body at different strengths of treatment.

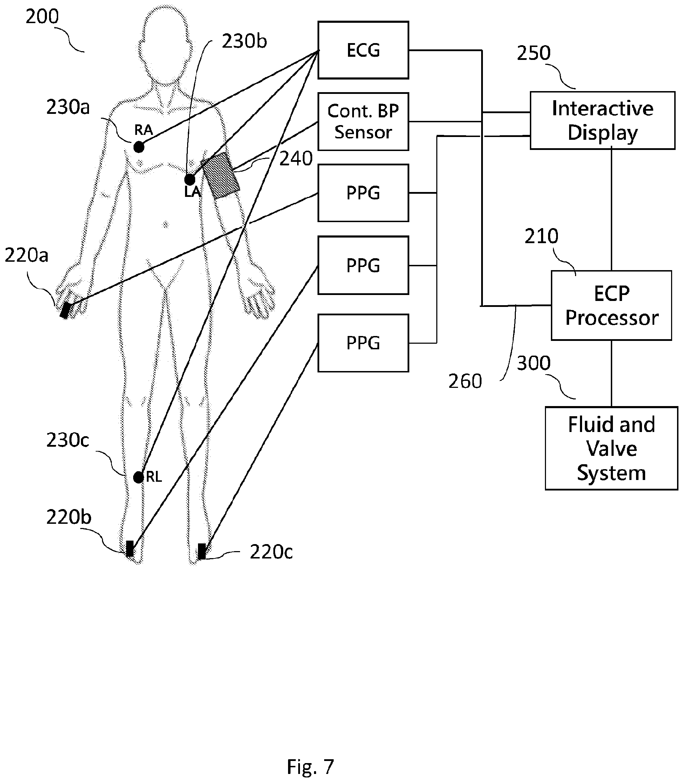

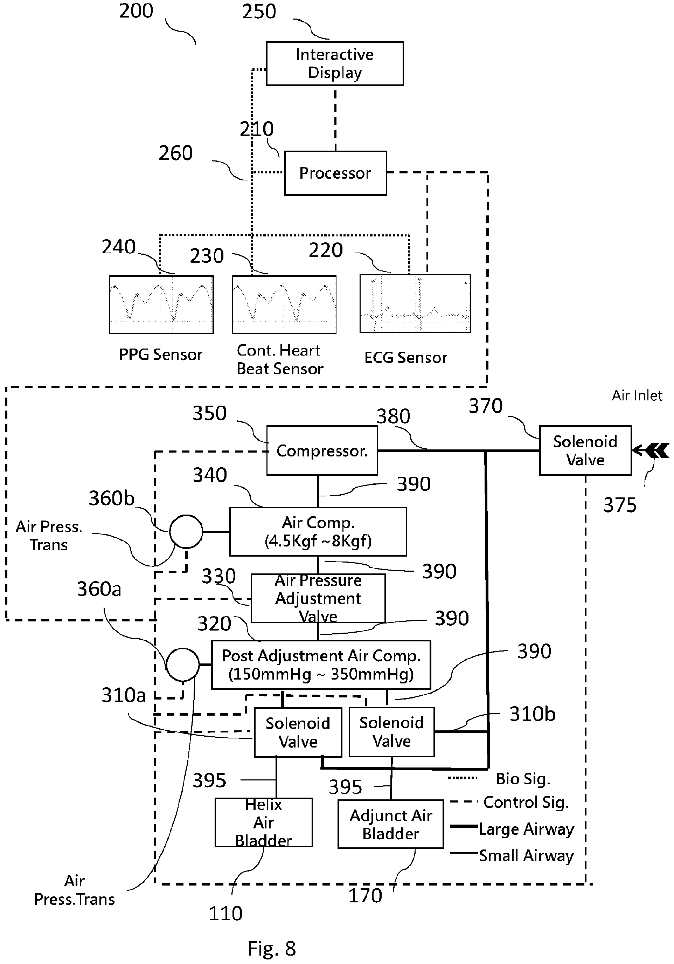

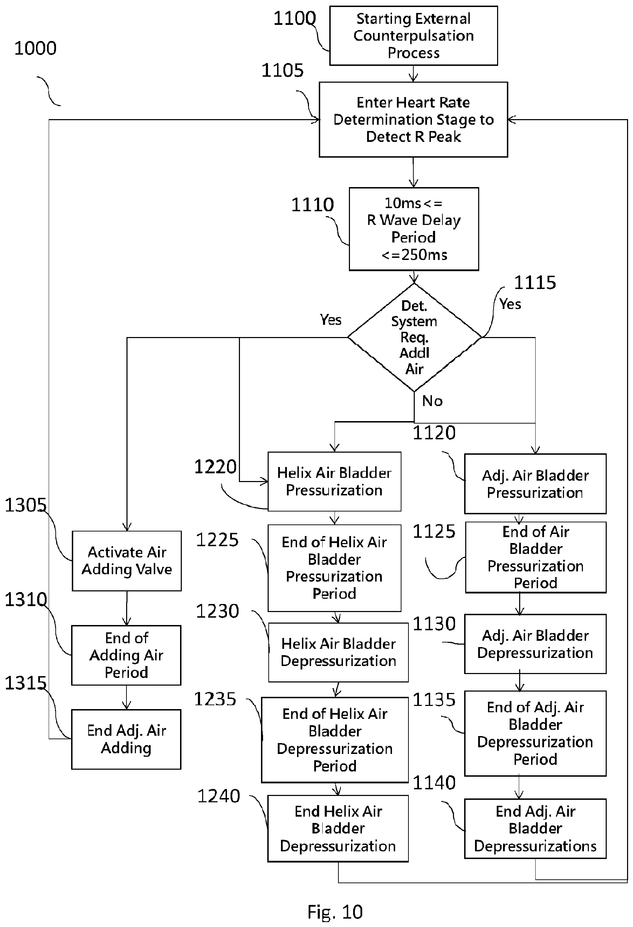

In an embodiment, as shown in figure 7, the ECP device 10 of the present invention further comprises an ECP controller system 200 configured to control various aspects of the ECP device 10 of the present invention including but not limited to interaction with the user, collection and analysis of user's biometric data and interaction with valve system 300 to pressurize/depressurize air bladders 110 and 170. In an embodiment, the ECP controller system 200 preferably comprises an ECP processor 210, one or more heartbeat sensors 220, 230, 240 and an interactive display unit 250. In an embodiment, the ECP processor 210 is connected to the interactive display unit 250, the heartbeat sensors 220, 230 and 240 via electronic connection 260. In addition, in an embodiment, the ECP processor 210 is further connected to a valve and fluid system 300 via electronic connection 260 as described in further details below in connection with figs. 8 and 10.

In an embodiment, the ECP processor 210 preferably comprises a processor configured to send, receive and process signals including but not limited to signals to and from the user via interactive display 250, signals to and from the valve and fluid system 300 as well as signals related to biometric data of the user such as heartbeat information collected by the heartbeat sensors 220, 230 and 240. In this way, the ECP processor 210 is configured to control various aspects of the ECP device 10 of the present invention such as pressure in the helix air bladder 110 and the adjunct air bladder 170 based on the various signals processed.

As shown in figure 7, in an embodiment, the PPG heartbeat sensor 220 may comprise a finger sensor 220a and two toe sensors 220b and 220c. In an embodiment, the ECG heartbeat sensor 230 may comprise a right and left chest sensor 230a and 230b. In addition, the ECG heartbeat sensor 230 may further comprise leg sensors 230c. In an embodiment, the continuous blood pressure sensor 240 comprises a blood pressure cuff over the arm of the user.

The display 250 is preferably a touchscreen that allows the user to interact with the ECP device 10 of the present invention such as triggering ECP treatment, input user information, system settings, etc.... User information may comprise biometric information of the user such as sex, height, weight, BMI, age, etc. Input information may also comprise systems settings such as type of ECP treatment and time period of treatment, maximum and/or minimum pressure, etc.... Output of information may comprise type of treatment, progress of treatment, etc....

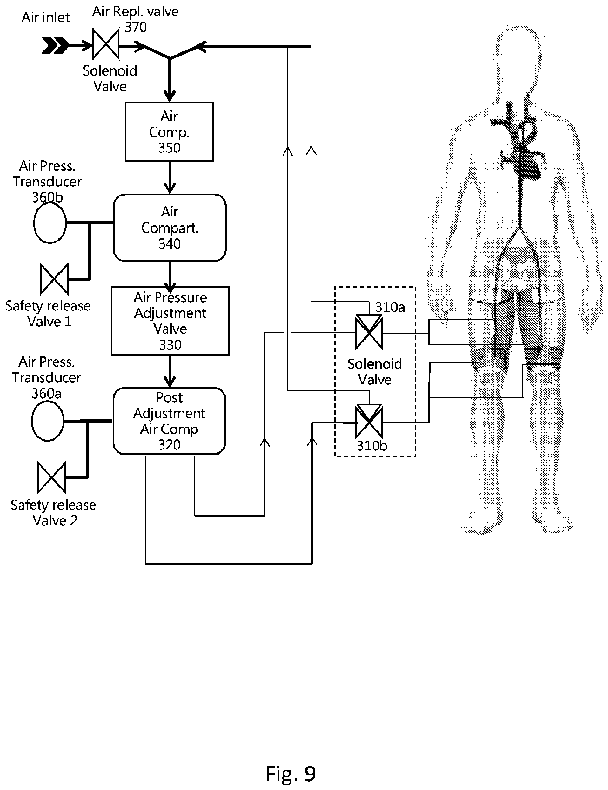

In an embodiment, as shown in Figs. 8 and 9, the ECP device of the present invention 10 further comprises a valve and fluid system 300 that works with the ECP controller system 200 to control pressure within the ECP device of the present invention 10, including pressure within the valve and fluid system 300 as well as the cuff system 100. In an embodiment, the valve and fluid system 300 comprises one or more air bladder valves 310, a post adjustment air compartment 320, an air pressure ratio adjustment valve 330, an air compressor air compartment 340, an air compressor 350, air pressure to electric signal transducer 360, air inlet valve 370, air inlet 375 and a series of large airways 380 and small airways 390 and 395. In an embodiment, the large airways 380, which are used for establishing negative pressure, have diameters of about 1 cm to 10 cm, and the small airways 390 and 395 have diameters of about 0.4 cm to 2 cm.

In an embodiment, the air bladder valves 310 each preferably comprises a valve configured to regulate pressure of the cuff system 100 based on electronic signals received from the ECP control system 100. In an embodiment, air bladder valves 310 are solenoid valves. The post adjustment air compartment 320 preferably comprises an air compartment capable of storing pressurized air for pressurizing the air bladders 110 and 170. In an embodiment, the pressure within the air compartment 320 is from 150 mmHg to 350 mmHg, 200 mmHg to 300 mmHg or about 250 mmHg. Each valve 310a and 310b is connected on one side to the post adjustment air compartment 320 via airway 390 and to helix air bladder 110 and adjunct air bladder 170 via airway 395 on the other side of the valve. Each valve 310 is additionally connected to air inlet valve 370 and compressor 350 via airway 380 through which air bladders 110 and 170 may be depressurized. Moreover, each valve 310 is electronically connected to ECP processor 210 via electronic connection 260 so that ECP processor 210 may electronically trigger valves 310 to pressurize and depressurize air bladders 110 and 170.

In an embodiment, the air compressor air compartment 340 comprises an air compartment that connects to the post adjustment air compartment 320 via the air pressure ratio adjustment valve 330. In an embodiment, the air pressure ratio adjustment valve 330 further connects to the ECP processor 210 via electronic connection 260. In this way, the air pressure ratio adjustment valve 330 is configured to maintain air pressure within the two air storages 320 and 340 based on signals from the ECP processor 210. In an embodiment, the air pressure in the post adjustment air storage 320 is maintained at between about from 150 mmHg to 350 mmHg, 200 mmHg to 300 mmHg, or about 250 mmHg while the air pressure within the compressor air storage 340 is maintained at about 4 kgf to 8 kgf, about 5 kgf to 7 kgf or about 6 kgf.

In an embodiment, the air compressor 350 comprises an air compressor configured to provide positive pressure to airway 390 when air inlet valve 370 is open and negative pressure to airway 380 when air inlet valve 370 is closed to air inlet 375 in order to facilitate replenishing air to the air compartment 340 and depressurizing the air bladders 110 and 170, respectively. In an embodiment, the air compressor 350 is capable of running at about 1700 rpm at about 130 L/m of flux at pressure up to about 8 kgf. The air inlet valve 370 preferably comprises a valve that connects to the compressor 350 via airway 380 on one end and to an air inlet 375 on the other end. In an embodiment, the air inlet valve 370 comprises a solenoid valve.

Lastly, transducers 360 preferably comprises transducers that each translates pressure to electric signal. Each transducer 360 preferably connects on one side to one of the air compartments 340 and 320, respectively, via airway 390 and to the ECP controller processor 210. In this way, the valve system 300 is configured to transmit air pressure information to ECP controller system 200 via transducers 360. As mentioned above, the ECP controller processor 210 is connected to solenoid valve 310, the air pressure ratio adjustment valves 330, air inlet valve 370 and air compressor 350 so that the ECP controller system 200 is configured to send electronic signals to control the valves 330 and 370 and air compressor 350 based on air pressure information from transducers 360a and 360b.

In an embodiment, when ECP processor 210 send a signal to valve 310, valve 310 pressurizes air bladders 110 and 170 by connecting them to post adjustment air compartment 320, supplying pressurized air to the air bladders 110 and 170. To depressurize air bladders 110 and 170, ECP processor 210 stops any signal to valve 310 so that valve 310 defaults to disconnecting the air bladders 110 and 170 from air compartment 320 and connecting them instead to airway 380. In addition, ECP processor 210 also sends a signal to air inlet valve 370 to close the air inlet so that compressor 350 is able to establish negative pressure in airway 380 to rapidly depressurize the air bladders 110 and 170. In an embodiment, the negative air pressure in airway 380 is about 80 mmHg to 120 mmHg, about 90 mmHg to 110 mmHg or about 100 mmHg.

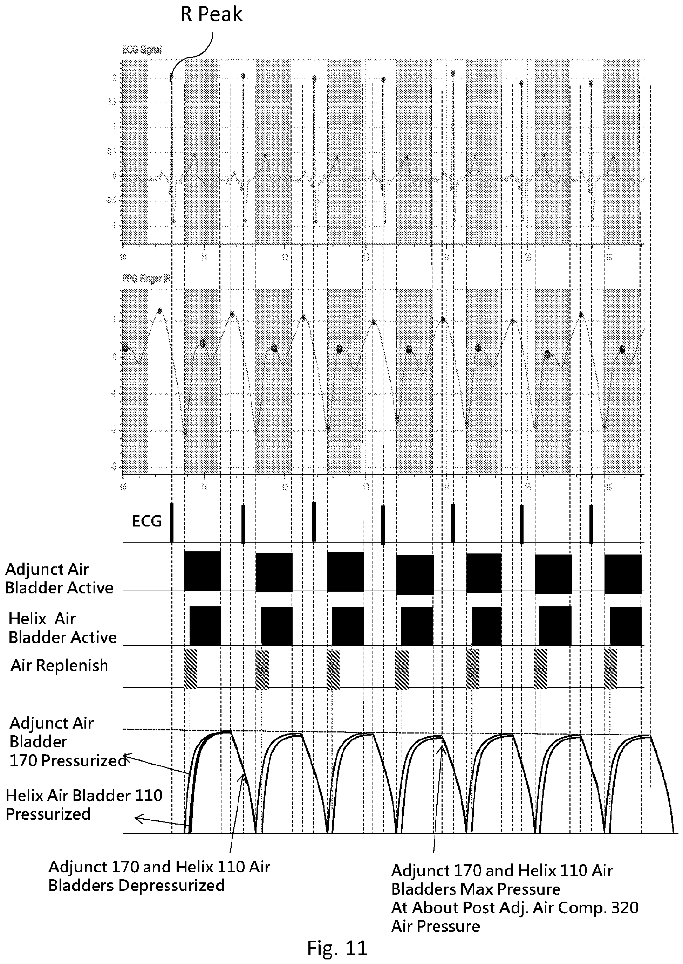

Figure 10 illustrates a method for providing External Counter Pulsation 1000. The method may be provided to treat diseases such as stroke, dementia, and arteriosclerosis but may also be provided merely to improve blood flow in general. As illustrated in fig. 11, in step 1100, the method is triggered to begin. In one embodiment, step 1100 may be manually triggered by a person such as the user via interactive display 250. In another embodiment, step 1100 may be triggered automatically by signals from the heart rate sensors 220, 230, 240. Next in step 1105, the ECP processor 210 reads and analyzes various biometric data such as but not limited to those input by the user via display as well as ECG 220, PPG 230, continuous blood pressure sensor 240, etc.... in order to determine the R peak of the user's heart rate. Once the R peak has been determined in step 1105 using various methods well known to persons in the art, the ECP processor 210 institutes a delay of between about 10 ms to about 250 ms, about 50 ms to about 200 ms or about 100 ms to about 150 ms from the R peak in step 1110. During the delay, in step 1115, a determination is made as to whether additional air is required in the valve and fluid system 300. In one embodiment, step 1115 is performed by the ECP processor 210 based on air pressure signals from transducers 360a and 360b which provide air pressure information for the air compartments 320 and 340, respectively. If in step 1115 it is determined that additional air is not required in either air compartments 320 and 340, for example, if the pressure within air compartments 320 and 340 is maintained between about 150 mmHg to 350 mmHg, 200 mmHg to about 300 mmHg, or about 250 mmHg, then no additional air is required, in step 1120, the ECP processor 210 triggers valve 310b to connect adjunct air bladder 170 to air compartment 320 to pressurize adjunct air bladder 170. In addition, in step 1220, the ECP processor 210 triggers valve 310a to connect helix air bladder 110 to air compartment 320 to pressurize helix air bladder 110 using air from air compartment 320. In an embodiment, step 1120 is performed before step 1220 wherein a delay of about 30 to 70 ms, or about 40 to 60 ms or about 50 ms is instituted between steps 1120 and 1220.

In step 1125, the end of the adjunct air bladder 170 pressurization period is reached. In an embodiment, the pressurization time period is about 200 ms to 600 ms, 250 ms to 550 ms, 300 ms to 500 ms or about 400 ms. In an embodiment, the pressurization time period maybe determined based upon heart rate according to the table below:

**Table 1**

| Heart Rate (per minute) | Pressurization Period (ms) |
|---|---|
| 105-120 | about 280 |
| 100-105 | about 300 |
| 95-100 | about 320 |
| 90-95 | about 340 |
| 85-90 | about 360 |
| 80-85 | about 380 |
| 75-80 | about 400 |
| 70-75 | about 440 |
| 60-65 | about 460 |
| 55-60 | about 480 |
| 50-55 | about 500 |
| <=50 | about 600 |

In an embodiment, the ECP processor 210 performs step 1125 by keeping track of this time period. Next in step 1130, the adjunct air bladder 170 is depressurized. In an embodiment, the depressurization is performed by the ECP processor 210 sending a signal to valve 310b to disconnect air bladder 170 from air compartment 320 to connect air bladder 170 to airway 380 as well as to close air inlet valve 370 to allow air compressor 350 to establish negative pressure in airway 380 to facilitate rapid depressurization of the adjunct air bladder 170. Next, in step 1135, the end of adjunct air bladder 170 depressurization period is reached. In an embodiment, the ECP processor 210 performs step 1135 by keeping account of this time period. In step 1140, adjunct air bladder 170 depressurization process is stopped and the process repeats from step 1105 if therapeutic effect has not been fully realized.

Similarly, after maintaining air pressure in the helix air bladder 110 for a preset time period the end of the helix cuff 105 pressurization period in step 1225. In an embodiment, the pressurization time period is about from 200 ms to 600 ms, 250 ms to 550 ms, 300 ms to 500 ms or about 400 ms. In another embodiment, the air pressure in the helix air bladder 110 is maintained according to the user's heart rate according to Table 1 minus any delay institute between steps 1120 and 1220 as discussed. In an embodiment, the ECP processor 210 performs step 1225 by keeping track of this time period. In step 1230, the helix air bladder 110 is depressurized. In an embodiment, the depressurization is performed by the ECP processor 210 sending a signal valve 310a to disconnect helix air bladder 110 from air compartment 320 to connect helix air bladder 110 to airway 380 in which negative pressure is established to depressurize the air bladder by closing air inlet valve 370 while compressor 350 is running. Next, in step 1235, the end of helix air bladder 110 depressurization period is reached. In an embodiment, the ECP processor 210 performs step 1235 by keeping track of this time period. In step 1240, the helix air bladder 110 depressurization process is stopped, and the process repeats from step 1105.

In an embodiment steps 1125 and 1225 are performed about the same time, and steps 1130 and 1230 are also performed about the same time so that both air bladders 110 and 170 are depressurized about the same time. In another embodiment, step 1125 is performed before step 1225, and step 1130 is performed before step 1230 so that the adjunct air bladder 170 is depressurized before the helix air bladder 110. In this embodiment, the delay is about from 20 ms to 100 ms, 30 ms to 90 ms, 40 ms to 80 ms or about 60 ms. In another embodiment, step 1125 is performed after step 1225, and step 1130 is performed after step 1230 so that the adjunct air bladder 170 is depressurized after the helix air bladder 110. In this embodiment, the delay is about from 20 ms to 100 ms, 30 ms to 90 ms, 40 ms to 80 ms or about 60 ms.

If in step 1115 ECP processor 210 determines that air replenishment is required in the air compartments 320 and 340, steps 1120 to 1140 and 1220 to 1240 are performed as described, but steps 1305 to 1315 are also performed to add more air into the system. Specifically, in step 1305, the ECP processor 210 signals valve 370 to open to air inlet 375 and ensures that compressor 350 is running to replenish air to air compartment 340. The air pressure ratio adjustment valve 330 in turn adds air to air compartment 320. In step 1310 as the system reaches end of air replenishment period, in an embodiment, the ECP processor 210 keeps track of the air replenishment period in step 1310. In step 1315, the ECP processor 210 sends signals to close air inlet valve 370 to stop adding air into the valve system. In an embodiment, since air bladder depressurization period requires that valve 370 to be closed so that negative pressure can be established in airway 380, steps 1305 to 1315 are performed concurrently with steps 1120 to 1125 and 1220 to 1225, before steps 1120 and 1220 or after steps 1140 and 1240 are completed.

Figs. 11 illustrate graphically the method 1000. As shown in fig. 11, the R peak is detected in step 1105 and a delay of about 10 ms to about 250 ms, about 50 ms to about 200 ms or about 100 ms to about 150 ms is instituted in step 1110 before the adjunct air bladder 170 is pressurized in step 1120. After the adjunct air bladder 170 is pressurized, the helix air bladder 110 is subsequently pressurized in step 1220 after a delay of about 50 ms. Also seen in figs. 11, if replenishment of air is required as determined in step 1115, it is done in steps 1305 to 1315 about the same time as the start of the adjunct air bladder 170 pressurization for about 50 ms to about 100 ms. Subsequently both the adjunct air bladder 170 and the helix air bladder 110 are depressurized at about the same time in steps 1125 to 1140 and in steps 1225 to 1240.

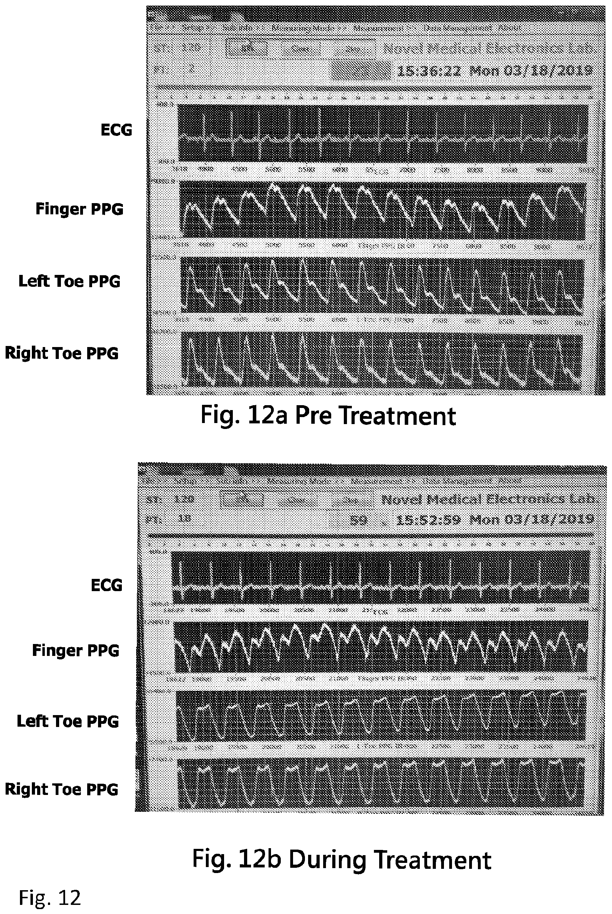

Fig. 12 illustrates the therapeutic effects of the ECP device 10 of the present invention. As seen in fig. 12a, prior to the treatment, PPG signal of a user is weak. During the treatment, the PPG signal of the user is much more regular and maintained at a constant strength as shown in figs. 12b.

It is to be understood that both the foregoing general description and the following detailed description are exemplary and explanatory only and are not restrictive of the invention, as claimed.

These and other changes can be made to the technology in light of the detailed description. In general, the terms used in the following disclosure should not be construed to limit the technology to the specific embodiments disclosed in the specification, unless the above detailed description explicitly defines such terms. Accordingly, the actual scope of the technology encompasses the disclosed embodiments and all the equivalent ways of practicing or implementing the technology.

## Claims

1. An External Counter Pulsation (ECP) Device (10) comprising:
a. an air bladder system (100) comprising one or more helix air bladders (110) and one or more adjunct air bladders (170) ;
b. a valve and fluid system (300) pneumatically connected to the air bladder system (100) wherein the valve and fluid system (300) is configured to pressurize and depressurize the helix air bladder (110) and the adjunct air bladders (170); and
c. a control system (200) comprising a processor (210) and one or more PPG sensors (220, 220a, 220b) and one or more ECG sensors (230, 230a, 230b, 230c) wherein the PPG and ECG sensors are connected to the user to collect PPG and ECG signals from the user and wherein the control system (200) is electronically connected to the valve and fluid system (300) to control the valve and fluid system (300) to pressurize or depressurize the air bladders (110, 170) of the air bladder system (100) based on signals detected by the sensors (220, 220a, 220b, 230, 230a, 230b, 230c) and wherein the adjunct air bladders (170) is positioned at either the lower end or the upper end of the helix air bladder (110) and is pressurized before the helix air bladder (110) so as to direct blood flow towards desired direction,
wherein each helix air bladder (110) is shaped such that, when attached to a user's thigh, the top end of the one or more helix air bladders (110) start from the front side of the thigh, wind around the femur in a helical fashion towards the inner thigh and the bottom end of the one or more helix air bladders (110) end at the rear side of the knee.

2. The ECP device (10) of claim 1 wherein helix air bladder's (110) length L (116) in cm is defined as height of the user/3.2 -b where b is between 15 cm to 30 cm, helix air bladder's (110) top width (112) and helix air bladder's bottom width (114) are each about 14 cm and helix angle (118) is about 55°.

3. The ECP device (10) of claim 1 wherein the wattage of the valve system (300) is less than about 1500 Watts.

4. The ECP device (10) of claim 1 wherein the helix air bladder (110) length L (116) does not exceed about 50 cm, W1 (112) and W2 (114) do not exceed about 25 cm and about 1250 cm² in area.

5. The ECP device (10) of claim 1 wherein the pressure within the helix air bladder (110) does not exceed about 350 mmHg when fully pressurized.

6. The ECP device (10) of claim 1 wherein the pressure of the helix air bladder (110) is not below about 150 mmHg when fully pressurized.

7. The ECP device (10) of claim 1 wherein the ratio of the top width (112) of the helix air bladder (110) W1 to the bottom width W2 (114) of the helix air bladder (110) is about from 1:1 to 2:1.

8. The ECP device (10) of claim 1 wherein the helix angle (118) of the helix air bladder (110) is between about 30° and 75°.

9. The ECP device (10) of claim 1 wherein the adjunct air bladders (170) is positioned at the lower end of the helix air bladder (110) with the adjunct air bladders (170) overlapping the helix air bladder (110).

10. The ECP device (10) of claim 1 wherein the adjunct air bladders (170) is positioned at the lower end of the helix air bladder (110) without the adjunct air bladders (170) overlapping the helix air bladder (110).

11. The ECP device (10) of claim 1 wherein the adjunct air bladder (170) is positioned at the upper end of the helix air bladder (110) with the adjunct air bladders (170) overlapping the helix air bladder (110).

12. The ECP device (10) of claim 1 wherein the adjunct air bladder (170) is positioned at the upper end of the helix air bladder (110) without the adjunct air bladders (170) overlapping the helix air bladder (110).

13. The ECP device (10) of claim 1 wherein the adjunct air bladder (170) and the helix air bladder (110) are in one single cuff.

## Patentansprüche

1. Externe Gegenpulsationsvorrichtung (ECP) (10) umfassend:
a. ein Luftbalgsystem (100), das eine oder mehrere Helix-Luftbälge (110) und eine oder mehrere Zusatz-Luftbälge (170) umfasst;
b. ein Ventil- und Fluidsystem (300), das pneumatisch mit dem Luftbalgsystem (100) verbunden ist, wobei das Ventil- und Fluidsystem (300) so konfiguriert ist, dass es den Helix-Luftbalg (110) und die Zusatz-Luftbälge (170) mit Druck beaufschlagt und druckentlastet; und
c. ein Steuerungssystem (200), das einen Prozessor (210) und einen oder mehrere PPG-Sensoren (220, 220a, 220b) und einen oder mehrere EKG-Sensoren (230, 230a, 230b, 230c) umfasst, wobei die PPG- und EKG-Sensoren mit dem Benutzer verbunden sind, um PPG- und EKG-Signale von dem Benutzer zu sammeln, und wobei das Steuerungssystem (200) elektronisch mit dem Ventil- und Fluidsystem (300) verbunden ist, um das Ventil- und Fluidsystem (300) zu steuern, um die Luftbälge (110, 170) des Luftbalgsystems (100) auf der Grundlage von Signalen, die von den Sensoren (220, 220a, 220b, 230, 230a, 230b, 230c) erfasst werden, unter Druck zu setzen oder den Druck zu verringern, und wobei der Zusatz-Luftbalg (170) entweder am unteren Ende oder am oberen Ende des Helix-Luftbalgs (110) angeordnet ist und vor dem Helix-Luftbalg (110) unter Druck gesetzt wird, um den Blutfluss in die gewünschte Richtung zu lenken,
wobei jeder Helix-Luftbalg (110) so geformt ist, dass, wenn er am Oberschenkel eines Benutzers angebracht ist, das obere Ende der einen oder mehreren Helix-Luftbälge (110) von der Vorderseite des Oberschenkels ausgeht, sich schraubenförmig um den Oberschenkel in Richtung des inneren Oberschenkels windet und das untere Ende der einen oder mehreren Helix-Luftbälge (110) an der Rückseite des Knies endet.

2. ECP-Vorrichtung (10) nach Anspruch 1, wobei die Länge L (116) des Helix-Luftbalgs (110) in cm definiert ist als Benutzergröße/3,2 - b, wobei b zwischen 15 cm und 30 cm liegt, die obere Breite (112) des Helix-Luftbalgs (110) und die untere Breite (114) des Helix-Luftbalgs jeweils etwa 14 cm betragen und der Steigungswinkel (118) etwa 55° beträgt.

3. ECP-Vorrichtung (10) nach Anspruch 1, wobei die Wattzahl des Ventilsystems (300) weniger als etwa 1500 Watt beträgt.

4. ECP-Vorrichtung (10) nach Anspruch 1, wobei die Länge L (116) des Helix-Luftbalgs (110) etwa 50 cm nicht überschreitet und W1 (112) und W2 (114) etwa 25 cm und eine Fläche von etwa 1250 cm² nicht überschreiten.

5. ECP-Vorrichtung (10) nach Anspruch 1, wobei der Druck in dem Helix-Luftbalg (110) bei vollem Druck etwa 350 mmHg nicht überschreitet.

6. ECP-Vorrichtung (10) nach Anspruch 1, wobei der Druck des Helix-Luftbalgs (110) nicht unter etwa 150 mmHg liegt, wenn er vollständig unter Druck steht.

7. ECP-Vorrichtung (10) nach Anspruch 1, wobei das Verhältnis der oberen Breite (112) des Helix-Luftbalgs (110) W1 zur unteren Breite W2 (114) des Helix-Luftbalgs (110) etwa 1:1 bis 2:1 beträgt.

8. ECP-Vorrichtung (10) nach Anspruch 1, wobei der Steigungswinkel (118) des Helix-Luftbalgs (110) zwischen etwa 30° und 75° liegt.

9. ECP-Vorrichtung (10) nach Anspruch 1, bei der die zusätzlichen Luftbälge (170) am unteren Ende des Helix-Luftbalgs (110) angeordnet sind, wobei die zusätzlichen Luftbälge (170) den Helix-Luftbalg (110) überlappen.

10. ECP-Vorrichtung (10) nach Anspruch 1, wobei die Zusatz-Luftbälge (170) am unteren Ende des Helix-Luftbalgs (110) angeordnet sind, ohne dass die Zusatz-Luftbälge (170) den Helix-Luftbalg (110) überlappen.

11. ECP-Vorrichtung (10) nach Anspruch 1, wobei der Zusatz-Luftbalg (170) am oberen Ende des Helix-Luftbalgs (110) positioniert ist und die Zusatz-Luftbälge (170) den Helix-Luftbalg (110) überlappen.

12. ECP-Vorrichtung (10) nach Anspruch 1, wobei der Zusatz-Luftbalg (170) am oberen Ende des Helix-Luftbalgs (110) positioniert ist, ohne dass die Zusatz-Luftbälge (170) den Helix-Luftbalg (110) überlappen.

13. ECP-Vorrichtung (10) nach Anspruch 1, bei der sich der Zusatz-Luftbalg (170) und der Helix-Luftbalg (110) in einer einzigen Manschette befinden.

## Revendications

1. Dispositif de contre-pulsation externe (ECP) (10) comprenant :
a. un système de vessies d'air (100) comprenant une ou plusieurs vessies d'air hélicoïdales (110) et une ou plusieurs vessies d'air complémentaires (170);
b. un système de valve et de fluide (300) raccordé, par voie pneumatique, au système de vessies d'air (100), dans lequel le système de valve et de fluide (300) est configuré pour mettre sous pression et dépressuriser la vessie d'air hélicoïdale (110) et les vessies d'air complémentaires (170); et
c. un système de commande (200) comprenant un processeur (210) et un ou plusieurs capteurs PPG (220, 220a, 220b) et un ou plusieurs capteurs ECG (230, 230a, 230b, 230c), dans lequel les capteurs PPG et ECG sont raccordés à l'utilisateur pour collecter des signaux PPG et ECG de l'utilisateur et dans lequel le système de commande (200) est électroniquement raccordé au système de valve et de fluide (300) pour commander le système de valve et de fluide (300) afin de mettre sous pression ou dépressuriser les vessies d'air (110, 170) du système de vessies d'air (100) sur la base des signaux détectés par les capteurs (220, 220a, 220b, 230, 230a, 230b, 230c) et dans lequel la vessie d'air complémentaire (170) est positionnée au niveau de l'extrémité inférieure ou de l'extrémité supérieure de la vessie d'air hélicoïdale (110) et est mise sous pression avant la vessie d'air hélicoïdale (110) afin de diriger l'écoulement de sang vers la direction souhaitée,
dans lequel chaque vessie d'air hélicoïdale (110) est formée de sorte que, lorsqu'elle est fixée à la cuisse de l'utilisateur, l'extrémité supérieure des une ou plusieurs vessies d'air hélicoïdales (110) commence à partir du côté avant de la cuisse, s'enroule autour du fémur d'une manière hélicoïdale vers l'intérieur de la cuisse et l'extrémité inférieure des une ou plusieurs vessies d'air hélicoïdales (110) se termine au niveau du côté arrière du genou.

2. Dispositif de ECP (10) selon la revendication 1, dans lequel la longueur L (116) de la vessie d'air hélicoïdale (110)en cm est définie comme étant la hauteur de l'utilisateur/3,2 - b, où b est compris entre 15 cm et 30 cm, la largeur supérieure (112) de la vessie d'air hélicoïdale (110) et la largeur inférieure (114) de la vessie d'air hélicoïdale (114) sont chacune d'environ 14 cm et l'angle d'hélice (118) est d'environ 55°.

3. Dispositif de ECP (10) selon la revendication 1, dans lequel la puissance électrique du système de valve (300) est inférieure à environ 1500 watts.

4. Dispositif de ECP (10) selon la revendication 1, dans lequel la longueur L (116) de la vessie d'air hélicoïdale (110) ne dépasse pas environ 50 cm, W1 (112) et W2 (114) ne dépassent pas environ 25 cm et environ 1250 cm² en surface.

5. Dispositif de ECP (10) selon la revendication 1, dans lequel la pression à l'intérieur de la vessie d'air hélicoïdale (110) ne dépasse pas environ 350 mmHg lorsqu'elle est complètement sous pression.

6. Dispositif de ECP (10) selon la revendication 1, dans lequel la pression de la vessie d'air hélicoïdale (110) n'est pas inférieure à environ 150 mmHg, lorsqu'elle est complètement sous pression.

7. Dispositif de ECP (10) selon la revendication 1, dans lequel le rapport de la largeur supérieure (112) de la vessie d'air hélicoïdale (110) W1 jusqu'à la largeur inférieure W2 (114) de la vessie d'air hélicoïdale (110) est d'environ 1/1 à 2/1.

8. Dispositif de ECP (10) selon la revendication 1, dans lequel l'angle d'hélice (118) de la vessie d'air hélicoïdale (110) est compris entre environ 30° et 75°.

9. Dispositif de ECP (10) selon la revendication 1, dans lequel les vessies d'air complémentaires (170) sont positionnées au niveau de l'extrémité inférieure de la vessie d'air hélicoïdale (110), avec les vessies d'air complémentaires (170) qui chevauchent la vessie d'air hélicoïdale (110).

10. Dispositif de ECP (10) selon la revendication 1, dans lequel les vessies d'air complémentaires (170) sont positionnées au niveau de l'extrémité inférieure de la vessie d'air hélicoïdale (110) sans que les vessies d'air complémentaires (170) ne chevauchent la vessie d'air hélicoïdale (110).

11. Dispositif de ECP (10) selon la revendication 1, dans lequel la vessie d'air complémentaire (170) est positionnée au niveau de l'extrémité supérieure de la vessie d'air hélicoïdale (110), avec les vessies d'air complémentaires (170) qui chevauchent la vessie d'air hélicoïdale (110).

12. Dispositif de ECP (10) selon la revendication 1, dans lequel la vessie d'air complémentaire (170) est positionnée au niveau de l'extrémité supérieure de la vessie hélicoïdale (110), sans que les vessies d'air complémentaires (170) ne chevauchent la vessie d'air hélicoïdale (110).

13. Dispositif de ECP (10) selon la revendication 1, dans lequel le vessie d'air complémentaire (170) et la vessie d'air hélicoïdale (110) sont dans un seul manchon.
